# DEMANDE DE BREVET EUROPEEN

(11) **EP 0 586 282 A1**
(43) Date de publication de la demande: **09.03.1994**
(21) Numéro de dépôt: 93402084.3
(22) Date de dépôt: 23.08.1993
(51) Int. Cl.: A61K 31/37, A61K 47/44

(54) **Compositions to piques comprenant du 5-méthoxypsoralène**

(30) Priorité: 03.09.1992 FR 9210523
(71) Demandeur: Goupil, Jean-Jacques, F-92100 Boulogne (FR)
(72) Inventeur: Goupil, Jean-Jacques, F-92100 Boulogne (FR)
(74) Mandataire: Petit, Hélène

(57) **Abrégé**

L'invention a pour objet des compositions pharmaceutiques applicables par voie topique, contenant du 5-méthoxypsoralène à une concentration de 60 ppm à 100 ppm, et plus particulièrement de 65 à 95 ppm, et de préférence 75 ppm, dans une formule huileuse pharmaceutiquement acceptable, ainsi que l'utilisation de ces compositions dans le cadre du traitement du psoriasis.

## Description

La présente invention a pour objet des compositions pharmaceutiques destinées au traitement par voie topique du psoriasis et d'autres dermatoses comprenant du 5-méthoxypsoralène (ou 5-MOP) à une concentration de 60 ppm à 100 ppm, dans une formule huileuse pharmaceutiquement acceptable.

Le psoriasis est une affection cutanée caractérisée par une hyper-prolifération des kératinocytes et un trouble de leur différenciation.

Il survient aussi une inflammation du derme, des modifications vasculaires et immunologiques mettant en jeu une activation des lymphocytes T.

Cette affection héréditaire, bénigne dans la grande majorité des cas, se manifeste par des plaques érythémato-squameuses plus ou moins étendues sur le revêtement cutané, provoquant une gène parfois importante pour le patient vis-à-vis de lui-même et de son entourage.

Souvent localisées aux coudes et avant-bras, aux genoux et jambes, à la région lombo-fessière et au cuir chevelu, les lésions peuvent s'étendre au reste du corps.

Parfois, le psoriasis par son extension très importante réalisant un psoriasis universalis ou même un psoriasis érythrodermique, par sa forme particulière pustuleuse ou par son association à un rhumatisme, réalise une affection très grave et invalidante pouvant mettre en jeu le pronostic vital ou fonctionnel du patient.

Le traitement du psoriasis par administration de 5-méthoxypsoralène par voie orale à des doses comprises entre 1 mg et 1,2 mg par kg de poids corporel, par séance de puvathérapie, et irradiation ultérieure aux UVA a fait l'objet du brevet français n° 2 406 444.

En raison des inconvénients liés aux puvathérapies générales prolongées, il est particulièrement intéressant de pouvoir disposer d'un traitement local sur une surface corporelle exposée aux UVA réduite.

La présente invention a précisément pour but de fournir une composition permettant d'éviter certains inconvénients du traitement par voie orale, et de procéder notamment à un traitement topique de psoriasis peu étendus ou de plaques rebelles ayant subsisté après un traitement par voie orale.

La présente invention a pour objet une composition pharmaceutique destinée à l'application par voie topique, caractérisée en ce qu'elle comprend du 5-méthoxypsoralène (5-MOP) à une concentration de 60 ppm à 100 ppm, et plus particulièrement de 65 ppm à 95 ppm, dans une formule huileuse pharmacologiquement acceptable.

Des compositions pharmaceutiques particulièrement préférées dans le cadre de la présente invention comprennent du 5-MOP à une concentration de l'ordre de 75 ppm.

On utilise généralement des essences naturelles de citrus comme l'essence de bergamote naturelle, qui contient du 5-méthoxypsoralène.

Les compositions pharmaceutiques de l'invention comprennent également un ou plusieurs filtres des rayons ultra-violet B, choisis notamment parmi le paraméthoxycinnamate d'éthyl-héxyle, le triméthylbenzylidène heptanone, etc.

Les compositions de l'invention se présentent dans un véhicule huileux ou sous forme d'émulsions à phase continue lipidique.

A titre illustratif, on peut citer la composition suivante qui se présente sous forme huileuse et est constituée notamment de:
. Huile d'arachide 68,970 %
. Myristate d'isopropyle 25,000 %
. ρ-méthoxycinnamate d'éthyl-2-hexyle 2,000 %
. 1.7.7-triméthyl-3-(méthyl-4-benzylidène) bicyclo (2.2.1)-2 heptanone 1,000 %
. Essence de bergamote naturelle q.s.p. 75 ppm en 5-MOP
. Essence de citrus synthétique q.s.p. 3 % 3,000 %
. Butylhydroxyanisole 0,020 %
. Butylhydroxytoluène 0,010 %

L'invention vise plus particulièrement l'utilisation d'essences naturelles de citrus apportant dans le produit fini une concentration en 5-MOP comprise entre 60 et 100 ppm, notamment entre 65 et 95 ppm, et plus particulièrement une concentration de 75 ppm, dans un véhicule huileux, pour l'obtention d'un médicament utile pour le traitement par voie topique du psoriasis et d'un certain nombre de dermatoses telles que le parapsoriasis, le vitiligo, l'eczéma atopique.

Les compositions selon l'invention sont appliquées sur les parties de la peau à traiter environ 30 mn à une heure avant l'irradiation de ces parties par des UVA. La dose initiale en UVA varie de 1 à 4 joules/cm² selon les phototypes de peau, la dose finale (pour les dernières séances de traitement) ne dépassant pas 8 joules/cm². Le traitement se poursuit généralement au rythme de 3 séances par semaine pendant 4 à 6 semaines.

On a ainsi traité trente patients, agés de plus de 18 ans, tous atteints de psoriasis localisé, en plaques, sans rémission depuis 6 mois, par la composition de l'invention contenant 75 ppm de 5-MOP décrite ci-dessus. Le traitement a duré 6 semaines, à raison de trois séances par semaine. La composition de l'invention était appliquée environ 30 minutes avant l'irradiation UVA en cabine DIXWELL. La dose d'UVA était augmentée de 0,5 joules toutes les deux séances, jusqu'à ce que le patient soit blanchi.

Il a été constaté que de façon remarquable, le traitement avec la composition selon l'invention, dosée à 75 ppm en 5-MOP, n'a entrainé aucun effet secondaire, aucun prurit, aucune irritation secondaire après l'application du produit, soit une tolérance parfaite chez 25 patients sur 30. Chez 5 patients, il est apparu un érythème persistant au cours des premières séances. Au niveau des résultats, sur les 30 cas traités, 25 psoriasis ont été blanchis complètement ou presque complètement, 4 ont subi une bonne amélioration et un une amélioration seulement légère.

Avec les compositions de l'invention dosées à 60 ppm en 5-MOP, les résultats de blanchiment complet ont été obtenus par des traitements s'étalant sur 4 à 10 semaines.

La mesure des améliorations obtenues se fait grâce à l'index de sévérité (qui prend en compte l'érythème, les squames et l'épaisseur de la plaque) qui permet d'apprécier la gravité du psoriasis et d'appréhender l'aspect clinique d'une amélioration.

Le traitement topique ci-dessus décrit peut être utilisé en complément de la puvathérapie orale dans laquelle une dose de 5-MOP de l'ordre de 1 mg/kg est administrée au patient environ deux heures avant l'irradiation. Ces traitements conjugués sont particulièrement recommandés pour les psoriasis étendus à plus de 40 % de la surface corporelle et pour faire disparaitre les plaques rebelles.

Il est intéressant de noter que l'association des traitements par voie topique et par voie orale, permet de diminuer le nombre de séances de puvathérapie de façon notable et qui peut être de 25%, cela dans l'intérêt évident du patient et de la santé publique.

## Revendications

1. Composition pharmaceutique destinée à l'administration topique, caractérisée en ce qu'elle contient comme substance active du 5-méthoxypsoralène à une concentration de 60 ppm à 100 ppm, et plus particulièrement de 65 ppm à 95 ppm, dans une formule huileuse pharmaceutiquement acceptable.

2. Composition pharmaceutique selon la revendication 1, caractérisée en ce qu'elle contient du 5-méthoxypsoralène à une concentration de 75 ppm, dans une formule huileuse pharmaceutiquement acceptable.

3. Composition pharmaceutique selon la revendication 1 ou la revendication 2, caractérisée en ce qu'elle comprend un ou plusieurs filtres des rayons ultra-violet B tels que le paraméthoxycinnamate d'éthyl-héxyle, le 1.7.7-triméthyl-3(méthyl-4-benzylidène)bicyclo(2.2.1)-2 heptanone.

4. Composition pharmaceutique selon l'une des revendications 1 à 3, caractérisée en ce qu'elle se présente sous forme huileuse et contient:
. Huile d'arachide 68,970 %
. Myristate d'isopropyle 25,000 %
. ρ-méthoxycinnamate d'éthyl-2-hexyle 2,000 %
. 1.7.7-triméthyl-3-(méthyl-4-benzylidène) bicyclo (2.2.1)-2 heptanone 1,000 %
. Essence de bergamote naturelle q.s.p. 75 ppm en 5-MOP
. Essence de citrus synthétique q.s.p. 3 % 3,000 %
. Butylhydroxyanisole 0,020 %
. Butylhydroxytoluène 0,010 %

5. Utilisation d'essences naturelles de citrus apportant dans le produit fini une concentration en 5-MOP comprise entre 60 ppm et 100 ppm, notamment entre 65 et 95 ppm, et plus particulièrement 75 ppm, en vue de réaliser un médicament destiné au traitement topique du psoriasis et d'un certain nombre d'autres dermatoses, telles que le parapsoriasis, le vitiligo, l'eczéma atopique.
